# EUROPÄISCHE PATENTSCHRIFT

(11) **EP 1 663 087 B1**
(45) Veröffentlichungstag und Bekanntmachung des Hinweises auf die Patenterteilung: **16.03.2011**
(21) Anmeldenummer: 05701074.6
(22) Anmeldetag: 20.01.2005
(51) Int. Cl.: A61F 9/008

(54) **STEUERUNG FÜR EINEN CHIRURGISCHEN LASER**
CONTROL DEVICE FOR A SURGICAL LASER
DISPOSITIF DE COMMANDE CONCU POUR UN LASER CHIRURGICAL

(30) Priorität: 23.01.2004 US 764311
(43) Veröffentlichungstag der Anmeldung: 07.06.2006
(73) Patentinhaber: Rowiak GmbH, 30419 Hannover (DE)
(72) Erfinder: LUBATSCHOWSKI, Holger, 30989 Gehrden (DE); RIPKEN, Tammo, 30459 Hannover (DE); OBERHEIDE, Uwe, 30982 Pattensen (DE)
(74) Vertreter: Eisenführ, Speiser & Partner
(86) Internationale Anmeldenummer: PCT/EP2005/000530
(87) Internationale Veröffentlichungsnummer: WO 2005/070358

(56) Entgegenhaltungen:
- EP-A- 0 903 133
- WO-A-2004/105661
- US-A1- 2002 049 450
- US-A1- 2004 199 149
- US-B1- 6 322 556

## Beschreibung

Die Erfindung betrifft das Gebiet der chirurgischen Laser und deren Steuerungen sowie Verfahren zur Behandlung eines transparenten Materials wie beispielsweise einer Augenlinse oder Augenhornhaut, insbesondere zur Behandlung von Presbyopie

Ab einem Alter von etwa 45 Jahren beginnt ein kontinuierliches Abnehmen der Akkommodationsfähigkeit der Linse des menschlichen. Auges. Dies äußert sich im Auftreten der Altersweitsichtigkeit (Presbyopie). Die Augenlinse ist aufgrund ihrer abnehmenden Elastizität nicht mehr in der Lage, sich so weit zu verdicken, wie es für ein scharfes Abbilden naher Objekte auf der Netzhaut notwendig ist. Von der abnehmenden Elastizität unberührt bleibt jedoch der Ziliarmuskel sowie der die Augenlinse umgebende Kapselsack, beide bleiben im Regelfalle aktiv und elastisch.

Krueger et al., Ophthalmology 108 (2001): 2123-2129 haben mit einem Neodym:YAG-Laser enukleirte menschliche Augenlinsen behandelt, indem sie durch Einstrahlen von Laserpulsen mit einer Pulsenergie von 2,5 bis 7,0 mJ ein ringförmiges Muster an Kavitationsblasen im Inneren der behandelten Linsen erzeugt haben. Sie konnten durch diese Behandlung eine Erhöhung der Linsen-Elastizität erreichen. Für eine wirksame Behandlung der Presbyopie ist es jedoch wünschenswert, die Elastizität weiter zu erhöhen. Zudem traten bei der Behandlung Kavitationsblasen, sowie darauffolgend langlebige Gasbläschen auf, die es erschwerten, die Änderung der Linsenbeugung zu messen. Ferner kam es zu einer für lebende Patienten nicht tolerierbaren Entwicklung von Stoßwellen und zu einer starken Erwärmung der Linse.

In der DE 199 40 712 A1 wird vorgeschlagen, zur Behandlung der Presbyopie im Inneren einer Augenlinse Bläschenfelder zu erzeugen, die durch Flüssigkeit gefüllt werden. Die Bläschen sollen das Linsenmaterial lockern und die Flexibilität der Linse erhöhen. Die Ergebnisse einer solchen Behandlung werden jedoch noch als unzureichend empfunden.

Eine Lasersteuerung gemäß dem Oberbegriff des unabhängigen Anspruchs 1 ist aus US 6322556B1 bekannt.

Aufgabe der vorliegenden Erfindung war es daher, ein verbessertes Verfahren zur Behandlung einer Augenlinse anzugeben, um die Elastizität der Linse zu erhöhen. Das Verfahren sollte insbesondere eine Behandlung der Presbyopie ermöglichen. Ferner sollte das Verfahren die Augenlinse schonen, insbesondere die Entwicklung starker Stoßwellen und großer Kavitationsblasen verringern.

Eine weitere Aufgabe der Erfindung war es, ein möglichst schonendes Verfahren bereitzustellen zum Behandeln eines transparenten Materials, insbesondere eines transparenten Materials biologischer Herkunft wie eine Augenhornhaut oder Augenlinse, um dessen Elastizität zu verbessern. Das Verfahren sollte möglichst wenig Zeit für eine Behandlung beanspruchen, das transparente Material sollte möglichst schonend behandelt werden und eine möglichst dauerhafte Verbesserung der Elastizität sollte erreicht werden. Zudem sollte das Verfahren insbesondere bei behandelten transparenten Materialien biologischer Herkunft ein rasches Abklingen und Verschwinden von durch die Behandlung eventuell hervorgerufenen Trübungen ermöglichen.

Eine weitere Aufgabe war es, Vorrichtungen zum Durchführen der aufgabenmäßigen Verfahren anzugeben.

Erfindungsgemäß wird deshalb eine Steuerung gemäß Anspruch 1 angegeben. Die Schnitte im transparenten Material werden vorzugsweise so ausgeführt, dass sie als Gleitebenen in verhärteten Bereichen einer Augenlinse dienen können.

Ferner wird erfindungsgemäß ein mit einer solchen Steuerung verbundener chirurgischer Laser selbst angegeben.

Die Erfindung wird nachfolgend mit Bezug auf die Behandlung einer Augenlinse beschrieben, anstelle einer Augenlinse können jedoch auch andere transparente Materialien behandelt werden, insbesondere eine Augenhornhaut. Die Begriffe "Augenlinse" und "transparentes Material" werden im Rahmen dieser Beschreibung gegeneinander austauschbar gebraucht, wobei eine Bezugnahme auf Augenlinse und Augenhornhäuten erfindungsgemäß besonders bevorzugt ist. Transparent im Sinne der vorliegenden Erfindung ist ein Material, das bei einer entsprechenden Wellenlänge einen Abschwächungskoeffizienten (Extinktionskoeffizient) von weniger als 100 pro cm, typischerweise weniger als 10 pro cm, hat.

Zudem wird erfindungsgemäß ein Verfahren angegeben zur Behandlung eines transparenten Materials, insbesondere einer Augenlinse und/oder Augenhornhaut, wobei im Inneren der Augenlinse bzw. des transparenten Materials eine Schnittfläche mit einer Mehrzahl an Laserpulsen erzeugt wird.

Die erfindungsgemäße Steuerung für einen vorzugsweise chirurgischen Laser ist dazu eingerichtet, einen Laser zu steuern, wenn ein solcher Laser mit der Steuerung verbunden wird. Die Steuerung kann dazu Mittel enthalten, um auf eine Laser-Lichtquelle steuernd einzuwirken, sie kann zusätzlich oder alternativ dazu auch Mittel enthalten, um auf Lichtleitmittel wie beispielsweise Spiegel zum Lenken von aus einer Laser-Lichtquelle ausgesandten Laserpulsen einzuwirken.

Im Folgenden schließt der Begriff "Laser" sowohl die eigentliche Laser-Lichtquelle als auch eventuell vorhandene Lichtleitmittel ein. Es versteht sich, dass die erfindungsgemäße Steuerung auch dann eingerichtet bleibt, einen Laser zu steuern, wenn zu einem gegebenen Zeitpunkt kein Laser mit der Steuerung verbunden sein sollte.

Die erfindungsgemäße Steuerung ermöglicht es, nach Eingabe eines entsprechenden Startsignals durch einen Benutzer einen gegebenenfalls mit der Steuerung verbundenen Laser automatisch ohne weitere Eingaben des Benutzers zu steuern, um das erfindungsgemäße Verfahren einschließlich eine seiner im Folgenden beschriebenen Varianten durchzuführen. Die Steuerung erleichtert es somit, die mit dem erfindungsgemäßen Verfahren einhergehenden Vorteile zu verwirklichen.

Durch das Erzeugen einer Schnittfläche im Inneren des zu behandelnden transparenten Materials (vorzugsweise einer Augenlinse und/oder einer Augenhornhaut) wird es erstmals ermöglicht, auf vorteilhaft einfache Weise die Elastizität des Materials stark zu erhöhen. Wenn eine Augenlinse oder Augenhornhaut behandelt wird, dann verändert die Schnittfläche Kraftlinien im Inneren der Augenlinse bzw. Augenhornhaut gegenüber ihrer Anordnung im unbehandelten Zustand der Augenlinse bzw. Augenhornhaut. Unter einer Kraftlinie einer Augenlinse wird dabei eine Trajektorie eines Volumenelements während eines Akkommodationsvorganges verstanden. In bevorzugten Ausführungsformen der erfindungsgemäßen Steuerung bzw. des erfindungsgemäßen Verfahrens steht eine Kraftlinie einer unbehandelten Augenlinse im Schnittpunkt mit einer Schnittfläche im wesentlichen senkrecht auf der Schnittfläche.

Die Schnittfläche kann plan oder gekrümmt sein. Die Schnittfläche kann eine Haupterstreckungsebene besitzen, die im wesentlichen senkrecht zur Haupteinstrahlrichtung des Lasers, im wesentlichen parallel oder auf andere Weise in einem Winkel zur Haupteinstrahlrichtung des Lasers ausgerichtet sein. Auf eine Augenlinse und Augehornhaut bezogen kann die Schnittfläche daher eine Haupterstreckungsebene besitzen, die frontal, sagittal oder in anderer Weise ausgerichtet ist.

Die Schnittfläche wird in dem transparenten Material, vorzugsweise der Augenlinse und/oder Hornhaut erzeugt durch eine Mehrzahl an Laserpulsen. Dies ermöglicht es, eine Schnittfläche beispielsweise im Inneren einer Augenlinse zu erzeugen, ohne dabei in den die Linse umgebenden Kapselsack, die Hornhaut und/oder die Oberfläche der Linse selbst einschneiden zu müssen. Der Schnitt kann deshalb auf besonders schonende Weise erzeugt werden. In bevorzugten Ausführungsformen der Erfindung wird das Verfahren daher so ausgeführt (bzw. ist die Steuerung so eingerichtet), dass der Kapselsack, die Hornhaut und/oder die Oberfläche der Linse nicht durchschnitten werden. Zusätzlich oder alternativ dazu kann das erfindungsgemäße Verfahren (bzw. die Steuerung) so eingerichtet sein, dass ein Schnitt im Inneren oder durch die Oberfläche einer Augenhornhaut erzeugt wird.

Die Laserpulse erzeugen beim Einstrahlen in eine Augenlinse oder Hornhaut Fehlstellen von weniger als 10 µm Durchmesser, vorzugsweise von 1-5 µm Durchmesser, in denen das faserige Material der Augenlinse oder Hornhaut zerstört ist. Bei einer Augenlinse oder Hornhaut sind die Fehlstellen mit Flüssigkeit der Augenlinse gefüllt. Eine Schnittfläche wird im Sinne dieser Erfindung gebildet durch eine Mehrzahl solcher Fehlstellen, die dicht genug beieinanderliegen, um eine zusammenhängende Fläche zu bilden, die insbesondere ein Aneinandervorbeigleiten der beiderseits der Schnittfläche liegenden Abschnitte des transparenten Materials ermöglicht. Im Unterschied dazu ermöglichen es herkömmliche lasergestützte Verfahren zur Behandlung von Augenlinsen nicht, Schnittflächen zu bilden; stattdessen wird lediglich die Beweglichkeit einzelner Kollagenfibrillen der Augenlinse verbessert, wie beispielsweise in der US 2004/0199149 A1 von Krueger und Myers beschrieben. In besonders bevorzugten Ausführungsformen des erfindungsgemäßen Verfahrens bzw. der erfindungsgemäßen Steuerung befinden sich im Inneren der Schnittfläche keine die beiden Seiten der Schnittfläche verbindenden Stege, vielmehr trennt eine Schnittfläche zwei benachbarte Abschnitte im Inneren des transparenten Materials (vorzugsweise der Augenlinse und/oder Augenhornhaut). Dies erleichtert insbesondere die Verformung der Augenlinse im Akkommodationsvorgang.

Die Laserpulse werden in bevorzugten Ausführungsformen der Erfindung so gesteuert, dass sie nach dem Durchgang durch den Bereich, in dem eine Schnittfläche erzeugt werden soll, aufgeweitet werden, um in Einstrahlrichtung hinter dem Bereich der Schnittfläche liegende Bereiche nicht zu verletzen. So kann bei der erfindungsgemäßen Behandlung eines Auges (bzw. mit der erfindungsgemäßen Steuerung) teilweise oder vollständig vermieden werden, empfindliche Bereiche der Netzhaut zu beschädigen oder zu verletzen.

In bevorzugten Ausführungsformen ist die erfindungsgemäße Steuerung eingerichtet, die Pulsenergie der Laserpulse auf einen Bereich von 1 pJ bis 1 µJ zu begrenzen. Das erfindungsgemäße Verfahren wird in bevorzugten Ausführungsformen entsprechend ausgeführt. Das Verfahren bzw. die wie soeben beschrieben eingerichtete erfindungsgemäße Steuerung ermöglichen es, im Vergleich zu herkömmlichen Verfahren sehr schwache Laserimpulse auf das zu behandelnde transparente Material (insbesondere eine zu behandelnde Augenlinse und/oder Augenhornhaut) einzustrahlen, so dass unerwünschte Stoßwellen und das Erzeugen von kataraktartigen Störungen in dem transparenten Material bzw. der Augenlinse weitgehend oder sogar vollständig vermieden werden können. Beides führt zu einer besonders schonenden und sicheren Behandlung des Auges. Besonders schonend behandelt wird eine Augenlinse und Augenhornhaut, wenn die Pulsenergie der Laserpulse auf einen Bereich von 1 pJ bis 500 nJ, vorzugsweise 100 pJ bis 100 nJ begrenzt wird. Vorzugsweise beträgt die Dauer eines Laserpulses weniger als 1 ps, besonders bevorzugt 1 fs bis 800 fs, insbesondere 50 fs-500 fs.

Ein Laserpuls mit einer Pulsenergie im Bereich von 1 pJ bis 1 µJ hinterlässt zudem in einer behandelten Augenlinse eine Fehlstelle mit einem Durchmesser von weniger als 10 µm, gewöhnlich von 1-5 µm. Mehrere solcher Laserpulse können daher, wenn sie in geeigneter Weise auf die zu behandelnde Augenlinse eingestrahlt werden, eine Schnittfläche mit einer kollateralen, laserinduzierten Veränderung des Augenlinsenmaterials in einer Dicke von weniger als 10 µm, insbesondere von 5 µm und insbesondere mit einer Dicke von 0,1-5 µm bilden wie oben beschrieben. Eine derart dünne Schnittfläche beeinträchtigt die Lichtdurchlässigkeit der behandelten Augenlinse nur sehr geringfügig und vermeidet zudem störende Verzerrungen.

Eine besonders bevorzugte Ausführungsform der erfindungsgemäßen Steuerung und entsprechend des erfindungsgemäßen Verfahrens ist zusätzlich oder alternativ zu den Merkmalen der übrigen Ausführungsformen eingerichtet, die Größe von durch die Laserpulse in der Augenlinse bzw. Augenhornhaut erzeugten Blasen auf Durchmesser höchstens 50 µm zu begrenzen. Blasen mit Durchmessern von mehr als 50 µm stören die Lichtdurchlässigkeit der Augenlinse und Augenhornhaut und gehen häufig einher mit starken Stoßwellen und mechanischen Beanspruchungen der nicht mit einem Laserpuls unmittelbar behandelten Bereiche der Augenlinse bzw. Augenhornhaut. Zudem kann es länger als einen Tag dauern, bis solche größeren Blasen mit Flüssigkeit gefüllt werden, bzw. bis sie kollabiert sind. Die erfindungsgemäße Ausführungsform (einschließlich des entsprechenden erfindungsgemäßen Verfahrens) vermeidet weitgehend oder sogar vollständig die soeben beschriebenen Nachteile. Dementsprechend ist es besonders bevorzugt, wenn der Durchmesser der erzeugten Blasen höchstens 30 µm beträgt, vorzugsweise 0,5-10 µm, besonders bevorzugt 0,5-1 µm. Wenn das in einer Blase enthaltene Gas entweicht und die Blase dadurch kollabiert, schrumpft ihr Durchmesser, bis sie sich schließlich zu einer oben beschriebenen Fehlstelle von weniger als, 10 µm Durchmesser, vorzugsweise weniger als 1 µm Durchmesser entwickelt.

Darüber hinaus ist eine solche Steuerung und ein entsprechendes Verfahren bevorzugt, die eingerichtet ist, die Schnittfläche durch zumindest 10.000 Laserpulsen, vorzugsweise durch zumindest 100.000 Laserpulsen, stärker bevorzugt zumindest 1.000.000 Laserpulsen, und besonders bevorzugt durch zumindest 10.000.000 Laserpulsen zu erzeugen. Durch die geschilderten hohen Zahlen an Laserpulsen können Schnittflächen mit besonders geringer Rauheit und besonders guter Glätte erzeugt werden. Dies führt zu einer besonders hohen gewonnenen Elastizität einer so behandelten Augenlinse.

Die erzeugte Schnittfläche besitzt vorzugsweise einen Flächeninhalt von 1 mm² bis 10 mm², besonders bevorzugt von 1 mm² bis 6 mm². Es hat sich gezeigt, dass Schnitte mit solchen Flächeninhaltett ausreichend sind, um eine deutlich erhöhte Elastizität einer behandelten Augenlinse zu erzielen.

Ferner ist es besonders bevorzugt, zwei aufeinanderfolgende Laserpulse so voneinander beabstandet zu erzeugen, dass von den Laserpulsen verursachte Fehlstellen im transparenten Material insbesondere in der Augenlinse und/oder Augenhornhaut einander nicht berühren oder überlappen. Unter aufeinander folgenden Laserpulsen werden dabei ein Paar Laserpulse verstanden, wobei in der Zeit zwischen dem ersten und zweiten Laserpuls kein weiterer Laserpuls erzeugt wird. Durch das räumliche Beabstanden der von einem solchen Laserpuls-Paar erzeugten Fehlstellen kann eine lokale Überbeanspruchung, insbesondere eine lokale Überhitzung und die Bildung unerwünscht großer Blasen mit mehr als 50 µm Durchmesser verhindert werden. Das vorstehend beschriebene erfindungsgemäße Verfahren und die entsprechend eingerichtete erfindungsgemäße Steuerung erlauben daher eine besonders schonende Behandlung einer Augenlinse.

Erfindungsgemäß ist es zum Herstellen von Schnittflächen in dem transparenten Material vorgesehen, den zum Erzeugen der Fehlstellen verwendeten Laserstrahl so auszurichten (bzw. durch eine erfindungsgemäße Steuereinrichtung so zu steuern), dass die Richtung des Laserstrahls im wesentlichen in oder tangential zur Schnittfläche liegt. Diese Ausrichtung des Laserstrahls ist insbesondere zum Erzeugen einer Schnittfläche in einer Augenlinse und/oder Augenhornhaut bevorzugt.

Dabei wird das Verfahren so durchgeführt (bzw. ist die Steuerung so eingerichtet), dass der Abstand zwischen in Strahlrichtung benachbarten Laserstrahl-Fokussierungspunkten eine

Rayleighlänge oder mehr beträgt, vorzugsweise jedoch weniger als vier Rayleighlängen. Durch das Fokussieren von Laserpulsen auf das transparente Material (insbesondere eine Augenlinse und/oder Augenhornhaut) werden im wesentlichen zylinderförmiger oder elliptischer Fehlstellen im transparenten Material gebildet, deren Längsachse in Laser-Strahlrichtung verläuft. Durch Einhalten zumindest einer Rayleighlänge, vorzugsweise jedoch vier oder weniger Rayleighlängen, zwischen dem Mittelpunkt zweier in Strahlrichtung benachbarter Fehlstellen wird sichergestellt, dass die Fehlstellen nicht miteinander verschmelzen, sondern einzeln bleiben. Die Fehlstellen können dann schneller als bei großen Fehlstellen kollabieren und mit Flüssigkeit gefüllt werden, so dass es insbesondere bei Behandlung einer Augenlinse und/oder Augenhornhaut rascher als bei Erzeugen großer, zusammenhängender Fehlstellen zum Verschwinden einer durch die Laserbehandlung vorübergehend hervorgerufenen Trübung der Augenlinse und/oder Augenhornhaut kommt. Zudem spart diese Art der Behandlung Zeit, da bezogen auf die gesamte Schnittfläche weniger Fehlstellen erzeugt werden müssen, als wenn die Schnittfläche weder tangential zur noch in der Laser-Strahlrichtung läge.

Gemäß einer nicht erfindungsgemäßen weiteren Ausführungsform ist es bevorzugt, den Laserstrahl so auszurichten, dass seine Strahlachse die zu erzeugende Schnittfläche durchstößt. Bei dieser Ausführungsform liegt die Strahlachse des Laserstrahls somit nicht in einer Schnittebene, sondern ist von dieser abgewinkelt. Vorteilhaft an dieser Ausführungsform ist, dass die von einem Laserpuls erzeugten zylinderförmigen oder elliptischen Fehlstellen nicht im wesentlichen mit ihren Längsachsen aufeinander folgen, sondern bezogen auf diese Längsachsen nebeneinander angeordnet sind. So wird auf vorteilhaft einfache Weise ein unbeabsichtigtes Verschmelzen zueinander benachbarter Fehlstellen vermieden so dass die erzeugten Fehlstellen schneller kollabieren und sich mit Flüssigkeit füllen, als wenn die Fehlstellen miteinander zu größeren Fehlstellen verschmolzen wären.

Die genannten Schnittführungen lassen sich neben Linsenmaterial auch auf anderes Material wie zum Beispiel die Hornhaut des Auges oder andere transparente Materialien anwenden. In der refraktiven Laserchirurgie des Auges oder der Keratoplastik kann die Minimierung von Gasbläschen und die Erhöhung der Schneidegeschwindigkeit von erheblichem Vorteil sein.

In einem erfindungsgemäßen Verfahren und einer entsprechenden erfindungsgemäßen Steuerung ist es zudem bevorzugt, eine Mehrzahl von Schnittflächen in vorgewählter.Anordnung zueinander zu erzeugen. Für jede einzelne Schnittfläche gilt dabei insbesondere das oben im Rahmen der erfindungsgemäßen Steuerung bzw. des erfindungsgemäßen Verfahrens Gesagte. Die Schnittflächen können beispielsweise einen Abschnitt der Augenlinse umgrenzen und somit die Fasern dieses Abschnitts vollständig von denen der übrigen Augenlinse trennen. Die Schnittflächen können ebenfalls beabstandete, einander nicht berührende oder durchschneidende Flächen bilden. Insbesondere können die Schnittflächen folgende Formen oder Teilflächen folgender Körper bilden: Kugel, Kugelabschnitt, Kugelausschnitt, Kugelschicht, Prismatoid oder Prisma mit elliptischer, ellipsenringförmiger, kreisförmiger, kreisringförmiger, parallelepipedförmiger, parallelogrammförmiger, rechteckiger, quadratischer, dreieckiger oder unregelmäßiger Grundfläche und Mantelfläche, wobei Grund- und Mantelfläche plan oder gekrümmt sein können.

In weiter bevorzugten Ausführungsformen des erfindungsgemäßen Verfahrens bzw. der entsprechend eingerichteten erfindungsgemäßen Steuerung werden zwei oder mehr Schnittflächen gleichzeitig erzeugt, indem abwechselnd Laserpulse zum Erzeugen der ersten und der zweiten Schnittfläche und gegebenenfalls der weiteren Schnittflächen erzeugt werden. Durch ein solches Vorgehen wird eine lokale Überhitzung und die Bildung unerwünscht großer Blasen mit mehr als 50 µm Durchmesser verhindert, ähnlich wie oben anhand einer einzigen Schnittfläche beschrieben.

Die erfindungsgemäße Steuerung bzw. das erfindungsgemäße Verfahren ist vorzugsweise so eingerichtet, dass eine oder eine Mehrzahl an Schnittflächen erzeugt werden, um die Akkommodationsfähigkeit einer Augenlinse auf zumindest 2 Dioptrien zu erhöhen, vorzugsweise auf zumindest 5 Dioptrien und besonders bevorzugt auf zumindest 10 Dioptrien zu erhöhen.

Die beschriebenen erfindungsgemäßen Verfahren können nicht-chirurgisch an Menschen oder Tieren entnommenem Material (ex vivo) durchgeführt werden, wobei das behandelte Material nach der Behandlung einem Menschen und/oder einem Tier implantiert werden kann.

Anhand der Figuren wird nachfolgend ein Ausführungsbeispiel der Erfindung näher beschrieben, ohne dass die Erfindung auf dieses Ausführungsbeispiel beschränkt werden soll. Es stellen dar:
- Fig. 1: eine Draufsicht auf eine erfindungsgemäß geschnittene Augenlinse;
- Fig. 2: eine Querschnittansicht der Augenlinse gemäß Fig. 1 entlang der Linie A-A;
- Fig. 3: eine Querschnittansicht der Augenlinse gemäß Fig. 1 entlang der Linie B-B; und
- Fig. 4: eine Querschnittansicht der Augenlinse gemäß Fig. 1 entlang der Linie C-C.
- Fig. 5: eine schematische Darstellung eines chirurgischen Lasers zur Behandlung der Augenlinse, insbesondere der Presbyopie

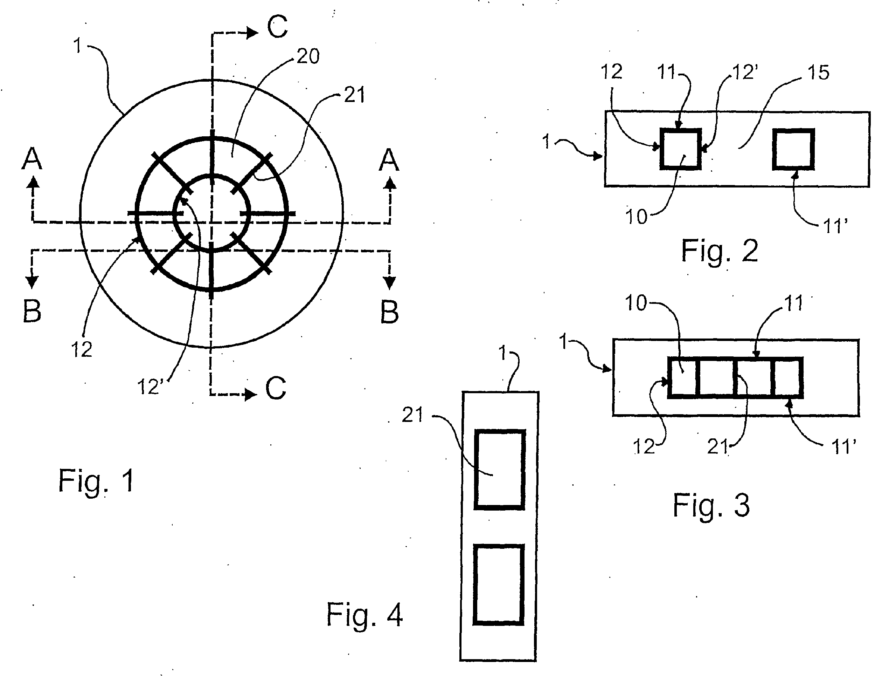

In Fig. 1 ist ein Ausschnitt einer Augenlinse 1 in schematischer Draufsicht dargestellt. Im Inneren der Augenlinse 1 ist ein Hohlzylinder 10 durch Schnittflächen 11, 11', 12, 12' von der übrigen Augenlinse abgetrennt. Die Figuren 2 bis 4 zeigen schematisch weitere Teilansichten des Hohlzylinders 10, wobei die Bezugszeichen aus Fig. 1 beibehalten wurden. Die Figuren 2 bis 4 zeigen lediglich diejenigen Schnitte und Formen, die auf den jeweiligen Linien A-A, B-B bzw. C-C liegen; auf eine räumliche Darstellung, in der auch weiter im Hintergrund liegende Details zu erkennen wären, wurde verzichtet.

Der Hohlzylinder 10 besitzt Deckenflächen 11, 11' in Form zweier kreisringförmiger Schnittflächen 11, 11'. Die Deckenflächen 11, 11' erstrecken sich im wesentlichen senkrecht zur Rotationsachse (nicht dargestellt) der Augenlinse 1. Die Deckenflächen 11, 11' sind zueinander im wesentlichen kongruent. Im Inneren der Deckenflächen 11, 11' ist jeweils ein kreisförmiger Bereich ausgespart, in dem keine Schnittfläche 11, 11' angeordnet ist.

Die Deckenflächen 11, 11' sind durch eine äußere Mantelfläche 12 und eine innere Mantelfläche 12' miteinander verbunden. Die Mantelflächen 12, 12' erstrecken sich jeweils ausgehend vom äußeren bzw. inneren Rand der kreisringförmigen Deckenflächen 11, 11' und stehen im wesentlichen senkrecht auf den Deckenflächen 11, 11'. Der von der inneren Mantelfläche 12' ummantelte Bereich 15 besitzt eine Mittelachse (nicht dargestellt), die im wesentlichen zusammenfällt mit der Rotationsachse der Augenlinse 1.

Senkrecht zur Mittelachse des Hohlzylinders 10 sind acht rechteckige Schnittflächen 21 angeordnet. Die Schnittflächen 21 zerteilen den Hohlzylinder 10 in acht voneinander getrennte Segmente 20. Die Schnittflächen 21 erstrecken sich in den Bereich 15 hinein und über den äußeren Rand der Deckenflächen 11, 11' bzw. der äußeren Mantelfläche 12 hinaus.

Zum Erzeugen des Hohlzylinders 10 und seiner Segmente 20 wird zunächst das zu behandelnde Auge auf den verwendeten Laser (nicht dargestellt) ausgerichtet. Der Laser ist mit einer Steuerung versehen, die das nachfolgend beschriebene Verfahren steuert.

Zunächst wird die Deckenflächen 11 und 11' gebildet. Dazu werden Laserpulse in die Augenlinse 1 eingestrahlt, so dass sich in der Ebene der Deckenflächen 11, 11' Fehlstellen bilden, an denen die Fasern der Augenlinse 1 durchtrennt sind. Die Laserpulse werden dabei so eingestrahlt, dass auf einen in die Ebene der Deckenfläche 11 gerichteten Laserpuls ein in die Ebene der Deckenfläche 11' gerichteter Laserpuls folgt. Somit sind die Laserpulse jedes Paar von Laserpulsen auf voneinander räumlich beabstandete Orte gerichtet, so dass die von ihnen verursachten Fehlstellen einander nicht berühren oder überlappen. Alternativ dazu kann auch zunächst die Deckenfläche 11 und anschließend die Deckenfläche 11' gebildet werden; auch in diesem Fall ist es zweckmäßig, die Laserpulse jedes Paar von Laserpulsen auf voneinander räumlich beabstandete Orte zu richten, so dass die von ihnen verursachten Fehlstellen einander nicht berühren oder überlappen.

Nachdem die Deckenflächen 11 und 11' gebildet wurden, werden auf entsprechende Weise die äußere Mantelfläche 12 und die innere Mantelfläche-12' gebildet. Schließlich werden noch die rechteckigen Schnittflächen 21 gebildet, um den Hohlzylinder 10 in einzelne Segmente 20 aufzuteilen.

Im Ergebnis wird durch die Zahl, Form und Anordnung der Schnittflächen 11, 11', 12, 12' und 21 die Elastizität der behandelten Augenlinse 1 so erhöht, dass diese eine Akkommodationsfähigkeit von zumindest 2 Dioptrien besitzt.

Als vorteilhaft hat sich in ersten Versuchen herausgestellt, in einem Bereich 15 um die Rotationsachse der Augenlinse 1 keine Schnittflächen vorzusehen. So wird erreicht, dass ein zentraler Bereich der Augenlinse 1 frei von Störungen bleibt.

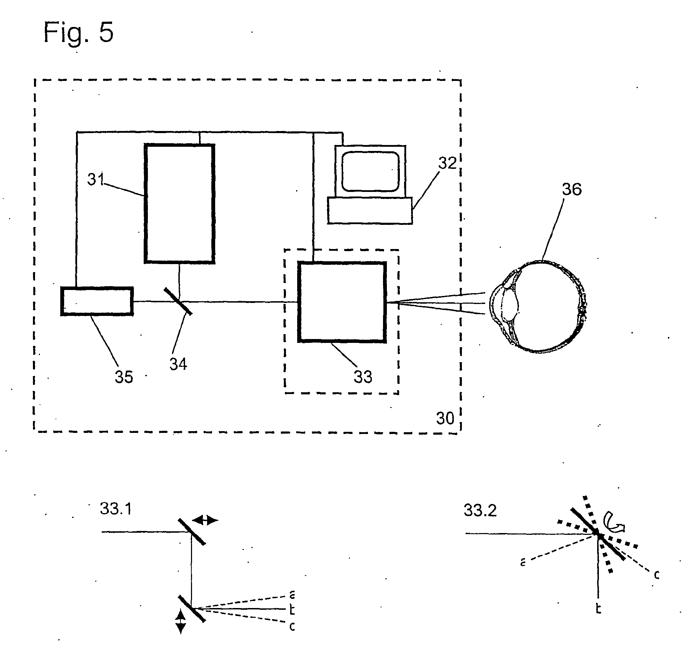

Der in Fig. 5 gezeigte chirurgische Laser 30 besitzt eine Laserstrahlquelle 31, beispielsweise einen Kurzpulslaser. Im Betrieb-geht von der Laserstrahlquelle 31 ein Laserstrahl aus, der über einen Umlenkspiegel 34 in einen Scanner 33 umgelenkt wird. Im Scanner 33 wird der Laserstrahl durch Verschieben weiterer Spiegel 33.1 und/oder Verschwenken weiterer Spiegel 33.2 in eine gewünschte Strahlrichtung abgelenkt und auf das zu behandelnde transparente Material ausgerichtet, beispielsweise auf ein Auge 36 mit einer zu behandelnden Augenlinse und/oder zu behandelnden Augenhornhaut. Ein Bildgebungsmittel 35 ist vorgesehen zum Überwachen der Tätigkeit des Lasers und des Fortgangs der Behandlung. Das Bildgebungsmittel 35 ist verbunden mit einem Steuerungsrechner 32. In Abhängigkeit von einem vorgewählten Behandlungsprogramm des transparenten Materials und vom erreichten Zustand der Behandlung wirkt der Steuerungsrechner 32 auf die Laserstrahlquelle 31 und den Scanner 33 ein, um die Behandlung gemäß dem vorgewählten Behandtungsprogramm vorzunehmen.

## Patentansprüche

1. Steuerung (32, 33) für einen Laser (30), die eingerichtet ist, einen mit der Steuerung verbindbaren Laser zu steuern, um im Inneren einer Hornhaut oder einer Augenlinse (1) eine Schnittfläche (21) mit einer Mehrzahl an Laserpulsen zu erzeugen, wobei
die Steuerung so eingerichtet ist, dass der Kapselsack, die Hornhaut und/oder die Oberfläche der Linse nicht durchschnitten werden, **dadurch gekennzeichnet, dass** die Steuerung eingerichtet ist, um die Laser-Strahlrichtung in die Ebene der zu erzeugenden Schnittfläche oder tangential zu der erzeugeneden Schnittfläche auszurichten und der Abstand zwischen in Strahlrichtung benachbarten Laserstrahl-Fokussierungspunkten eine Rayleighlänge oder mehr beträgt.

2. Steuerung nach Anspruch 1, wobei die Steuerung eingerichtet ist, die Pulsenergie der Laserpulse auf einen Bereich von 1 pJ bis 1 µJ zu begrenzen.

3. Steuerung nach einem der vorherigen Ansprüche, wobei die Steuerung eingerichtet ist, die Schnittfläche durch zumindest 10000 Laserpulsen zu erzeugen.

4. Steuerung nach einem der vorherigen Ansprüche, wobei die Steuerung eingerichtet ist, die Schnittfläche mit einem Flächeninhalt von 1 mm² bis 10 mm² zu erzeugen.

5. Steuerung nach einem der vorherigen Ansprüche, wobei die Steuerung eingerichtet ist, zwei aufeinanderfolgende Laserpulse so voneinander beabstandet anzuordnen, dass von den Laserpulsen verursachte Fehlstellen in dem transparenten Material einander nicht berühren oder überlappen.

6. Steuerung nach einem der vorherigen Ansprüche, wobei die Steuerung eingerichtet ist, den Laser zu steuern, um eine Mehrzahl der Schnittflächen in vorgewählter Anordnung zueinander zu erzeugen.

7. Chirurgischer Laser (30), wobei der Laser in Steuerverbindung steht mit einer Steuerung (32) nach einem der Ansprüche 1 bis 6.

8. Verfahren zur ex vivo Bearbeitung eines transparenten Materials, wobei im Inneren des Materials eine Schnittfläche mit einer Mehrzahl an Laserpulsen erzeugt wird und die Oberfläche des Materials nicht durchschnitten wird, **dadurch gekennzeichnet dass** die Strahlrichtung des Lasers (30) in die Ebene der zu erzeugenden Schnittfläche oder tangential zu der zu erzeugenden Schnittfläche ausgerichtet wird und der Abstand zwischen in Strahlrichtung benachbarten Laserstrahl-Fokussierungspunkten eine Rayleighlänge oder mehr beträgt.

9. Verfahren nach Anspruch 8, wobei die Pulsenergie der Laserpulse auf einen Bereich von 1 pJ bis 1 µJ begrenzt wird.

10. Verfahren nach einem der Ansprüche 8 oder 9, wobei die Schnittfläche durch zumindest 10000 Laserpulsen erzeugt wird.

11. Verfahren nach einem der Ansprüche 8 bis 10, wobei die Schnittfläche mit einem Flächeninhalt von 1 mm² bis 10 mm² erzeugt wird.

12. Verfahren nach einem der Ansprüche 8 bis 11, wobei zwei aufeinanderfolgende Laserpulse so voneinander beabstandet erzeugt werden, dass von den Laserpulsen verursachte Fehlstellen in dem transparenten Material einander nicht berühren oder überlappen.

13. Verfahren nach einem der Ansprüche 8 bis 12, wobei eine Mehrzahl der Schnittflächen in vorgewählter Anordnung zueinander erzeugt werden.

## Claims

1. Control device (32,33) for a laser (30), which control device (32, 33) is set up to control a laser that can be connected to the control device, in order to generate within a cornea or an eye lens (1) a cut surface (21) with a plurality of laser pulses, wherein the control device is set up in such a way that the capsular bag, the cornea and/or the surface of the lens are not intersected, **characterised in that** the control device is set up in order to align the laser beam direction in the plane of the cut surface to be generated or tangentially to the cut surface to be generated and the distance between adjacent laser beam focusing points in the laser beam direction is one or more Rayleigh length(s).

2. Control device according to claim 1, wherein the control device is set up to limit the pulse energy of the laser pulses to a range of 1 pJ to 1 µJ.

3. Control device according to one of the preceding claims, wherein the control device is set up to generate the cut surface by at least 10,000 laser pulses.

4. Control device according to one of the preceding claims, wherein the control device is set up to generate the cut surface with a surface area of 1 mm² to 10 mm².

5. Control device according to one of the preceding claims, wherein the control device is set up to arrange two successive laser pulses with a separation from one another such that blemishes, caused by the laser pulses, in the transparent material do not touch or overlap with each other.

6. Control device according to one of the preceding claims, wherein the control device is set up to control the laser in order to generate a plurality of the cut surfaces in a preselected arrangement with each other.

7. Surgical laser (30), wherein the laser has a control connection with a control device (32) according to one of claims 1 to 6.

8. Method for ex vivo processing of a transparent material, wherein within the material a cut surface is generated with a plurality of laser pulses and the surface of the material is not intersected, **characterised in that** the beam direction of the laser (30) is aligned in the plane of the cut surface to be generated or tangentially to the cut surface to be generated and the distance between adjacent laser beam focusing points in the laser beam direction is one or more Rayleigh length(s).

9. Method according to claim 8, wherein the pulse energy of the laser pulses is limited to a range of 1 pJ to 1 µJ.

10. Method according to one of claims 8 or 9, wherein the cut surface is generated by at least 10,000 laser pulses.

11. Method according to one of claims 8 to 10, wherein the cut surface is generated with a surface area of 1 mm² to 10 mm².

12. Method according to one of claims 8 to 11, wherein two successive laser pulses are generated with a separation from one another such that blemishes, caused by the laser pulses, in the transparent material do not touch or overlap with each other.

13. Method according to one of claims 8 to 12, wherein a plurality of the cut surfaces is generated in a preselected arrangement to one another.

## Revendications

1. Dispositif de commande (32, 33) pour un laser (30) qui est conçu pour commander un laser pouvant être raccordé au dispositif de commande, pour produire, à l'intérieur d'une cornée ou d'un cristallin (1), une section (21) avec une multiplicité d'impulsions laser,
le dispositif de commande étant conçu de telle sorte que le sac capsulaire, la cornée et/ou la surface supérieure du cristallin ne soient pas sectionnés, et **caractérisé en ce que** le dispositif de commande est conçu pour orienter la direction du faisceau laser dans le plan de la section à produire ou tangentiellement à celui-ci et la distance entre des points de focalisation du faisceau laser voisins dans le faisceau est une longueur de Raleigh ou plus.

2. Dispositif de commande selon la revendication 1, dans lequel le dispositif de commande est conçu pour limiter l'énergie d'impulsion de l'impulsion laser à une plage de 1 pJ à 1 µJ.

3. Dispositif de commande selon l'une des revendications précédentes, dans lequel le dispositif de commande est conçu de telle sorte que la section soit produite par au moins 10000 impulsions laser.

4. Dispositif de commande selon l'une des revendications précédentes, dans lequel le dispositif de commande est conçu pour générer la section avec une surface de 1 mm² à 10 mm².

5. Dispositif de commande selon l'une des revendications précédentes, dans lequel le dispositif de commande est conçu pour disposer à distance d'une de l'autre, deux impulsions laser consécutives, de telle sorte que des sites manqués provoqués par les impulsions laser dans le matériau transparent ne se touchent ou ne se chevauchent pas.

6. Dispositif de commande selon l'une des revendications précédentes, dans lequel le dispositif de commande est conçu pour commander le laser pour générer une multiplicité de sections dans une disposition prédéfinie.

7. Laser chirurgical (30), dans lequel le laser est en communication de commande avec un dispositif de commande (32) selon l'une des revendications 1 à 6.

8. Procédé de traitement *ex vivo* d'un matériau transparent, suivant lequel, à l'intérieur du matériau, une section est produite avec une multiplicité d'impulsions laser et la surface supérieure du matériau n'est pas sectionnée, **caractérisé en ce que** la direction du faisceau du laser (30) est orientée dans le plan de la section à produire ou tangentiellement à celui-ci et la distance entre des points de focalisation du faisceau laser voisins dans le faisceau est une longueur de Raleigh ou plus.

9. Procédé selon la revendication 8, dans lequel l'énergie d'impulsion de l'impulsion laser est limitée à une plage de 1 pJ à 1 µJ.

10. Procédé selon l'une des revendications 8 ou 9, dans lequel la section est générée par au moins 10000 impulsions laser.

11. Procédé selon l'une des revendications 8 à 10, dans lequel la section est produite avec une surface de 1 mm² à 10 mm².

12. Procédé selon l'une des revendications 8 à 11, dans lequel deux impulsions laser consécutives sont produites à distance l'une de l'autre de telle sorte les sites manqués par les impulsions laser dans le matériau transparent ne se touchent ou ne se chevauchent pas.

13. Procédé selon l'une des revendications 8 à 12, dans lequel une multiplicité de sections est produite dans un ordre prédéterminé.
